# EUROPÄISCHE PATENTSCHRIFT

(11) **EP 1 035 906 B1**
(45) Veröffentlichungstag und Bekanntmachung des Hinweises auf die Patenterteilung: **16.06.2010**
(21) Anmeldenummer: 98965184.9
(22) Anmeldetag: 26.11.1998
(51) Int. Cl.: B01D 53/84, C02F 3/32, C02F 9/00

(54) **VERFAHREN ZUR REDUZIERUNG DER KONZENTRATION VON INHALTSSTOFFEN IN EINEM GAS UND IN EINER FLÜSSIGKEIT UND VORRICHTUNG ZUR DURCHFÜHRUNG DIESES VERFAHRENS**
METHOD AND DEVICE FOR REDUCING THE CONCENTRATION OF INGREDIENTS IN A GAS AND IN A LIQUID
PROCEDE DE REDUCTION DE LA CONCENTRATION DE SUBSTANCES CONTENUES DANS UN GAZ ET DANS UN LIQUIDE, ET DISPOSITIF POUR LA MISE EN OEUVRE DE CE PROCEDE

(30) Priorität: 27.11.1997 DE 19752542
(43) Veröffentlichungstag der Anmeldung: 20.09.2000
(73) Patentinhaber: Zech Umwelt GmbH, 27777 Ganderkesee (DE)
(72) Erfinder: STELLING, André, D-27367 Sottrum (DE); RICHERT, Olaf, D-28857 Syke-Barrien (DE)
(74) Vertreter: Eisenführ, Speiser & Partner
(86) Internationale Anmeldenummer: PCT/EP1998/007617
(87) Internationale Veröffentlichungsnummer: WO 1999/028018

(56) Entgegenhaltungen:
- WO-A-98/22201
- DE-A- 3 108 923
- DE-A- 3 607 864
- DE-A- 4 419 766
- DE-C- 4 444 191
- DATABASE WPI Section Ch, Week 9310 Derwent Publications Ltd., London, GB; Class D16, AN 93-079582 XP002096898 & JP 05 023541 A (EBARA CORP ET AL) , 2. Februar 1993
- YUN Y -S ET AL: "CARBON DIOXIDE FIXATION BY ALGAL CULTIVATION USING WASTEWATER NUTRIENTS" JOURNAL OF CHEMICAL TECHNOLOGY AND BIOTECHNOLOGY (INTERNATIONAL JOURNAL OF BIOTECHNICAL AND CHEMICAL PROCESSES), Bd. 69, Nr. 4, August 1997, Seiten 451-455, XP000702347
- STOCKHAMMER S ET AL: "EINSATZ VON BIOWAESCHERN IN DER CHEMISCHEN PRODUKTION" CHEMIE. INGENIEUR. TECHNIK, Bd. 64, Nr. 2, 1. Februar 1992, Seiten 148-155, XP000257547
- PATENT ABSTRACTS OF JAPAN vol. 015, no. 409 (C-0876), 18. Oktober 1991 & JP 03 169324 A (MITSUBISHI HEAVY IND LTD), 23. Juli 1991

## Beschreibung

Die Erfindung betrifft ein Verfahren zur Reduzierung der Inhaltsstoffe Ammoniak, Phosphat, Ammonium, Nitrat, Nitrit, Amine, Schwefelwasserstoff, Alkohole, Ketone, Ester, organische Säuren, organische Schwefelverbindungen, aromatische Kohlenwasserstoffe und sonstige Kohlenwasserstoffverbindungen und/oder Kohlendioxid in der Abluft und im Prozesswasser einer Anlage.

Des Weiteren betrifft die Erfindung eine Vorrichtung zur Reduzierung der Inhaltsstoffe Ammoniak, Phosphat, Ammonium, Nitrat, Nitrit, Amine, Schwefelwasserstoff, Alkohole, Ketone, Ester, organische Säuren, organische Schwefelverbindungen, aromatische Kohlenwasserstoffe und sonstige Kohlenwasserstoffverbindungen und/oder Kohlendioxid in der Abluft und im Prozesswasser einer Anlage, insbesondere zur Durchführung eines Verfahrens der zuvor genannten Art.

Es sind Verfahren und Vorrichtungen bekannt, die zur Reinigung von mit verschiedenen Inhaltsstoffen belasttem Gas, insbesondere Abluft aus landwirtschriftlichen oder industriellen Betrieben, eingesetzt werden. Hierbei wird beispielsweise als Wäschereinheit ein Rieselbettreaktor eingesetzt, in welchem eine Flüssigkeit mit Inhaltsstoffen des Gases direkt in Berührung kommt und diese bindet. Außerdem benetzt die Flüssigkeit in den Rieselbettreaktoren gefüllte Füllkörper, auf deren großen Oberflächen heterotrophe Mikroorganismen in natürlicher Weise wachsen. Die Mikroorganismen binden Inhaltsstoffe aus dem Gas und mineralisieren sie. Die mineralisierten Stoffe reichern sich dann in der Flüssigkeit an, die anschließend aus dem Rieselbettreaktor und beispielsweise zurück zur Berieselungseinrichtung des Reaktors geleitet wird. Ist die Flüssigkeit mit der Zeit jedoch zu sehr mit Inhaltsstoffen aus dem Gas bzw. mit mineralisierten Stoffen von den Mikroorganismen beladen, wird sie fortgeleitet und neue Flüssigkeit dem Rieselbettreaktor zugeführt. Nachteilig bei diesem bekannten Verfahren bzw. bei dieser bekannten Vorrichtung ist, daß die Flüssigkeit ungereinigt bleibt und daher einerseits meistens nicht wiederverwertbar und evtl. nur schwer bzw. kostenintensiv entsorgbar ist und andererseits nicht beliebig oft durch den Rieselbettreaktor geleitet werden kann, wodurch Aufwand und Kosten steigen.

Die DE 31 08 923 A1 offenbart ferner ein anaerobes Aufschlussverfahren, wodurch Biogas zum Zwecke der Erhöhung des Methananteils im Biogas durch Reduzierung des Schwefelwasserstoff- und Kohlendioxidanteils gereinigt wird. Demnach ist Biogas in diesem Fall als Wertstoff zu betrachten, dessen Qualität durch dieses bekannte Verfahren erhöht werden soll. Ferner wird Fermentationswasser durch Zugabe von Calciumhydroxid alkalisiert, wobei also ein Stoff hinzugegeben wird, der von Algen nicht oder nur in geringem Maße aufgenommen wird und zu einer elementaren Veränderung der Abwasserbeschaffenheit führt. Schließlich wird bei diesem bekannten Verfahren in der Flüssigkeit der BSB (Biochemischer Sauerstoff-Bedarf) reduziert, der sich aus dem Sauerstoff von heterotrophen Mikroorganismen im Wasser ergibt.

Die DE 44 19 766 C2 beschreibt ebenfalls ein Verfahren zur biologischen Reinigung von Biogasen und Anreicherung von Methan. Bei diesem bekannten Verfahren wird Schwefelwasserstoff und Kohlendioxid mit einem Lösungsmittel aus dem Biogas entfernt, das Methan abgetrennt und anschließend örtlich getrennt davon das Lösungsmittel regeneriert. Außerdem wird bei diesem bekannten Verfahren das zu reinigende Biogas direkt mit Algen in Berührung gebracht.

Die JP 03169324 A schlägt vor, Meerwasser als Waschflüssigkeit zu verwenden.

Aufgabe der vorliegenden Erfindung ist es, ein Verfahren sowie eine Vorrichtung der eingangs genannten Art zur Verfügung zu stellen, um die eingangs genannten Inhaltsstoffe in der Abluft und im Prozesswasser einer Anlage in einem kombinierten Verfahren auf einfache und kostengünstige Weise entfernen zu können, ohne möglichst die Umwelt zu belasten.

Diese Aufgabe wird gemäß einem ersten Aspekt der Erfindung gelöst durch ein Verfahren zur Reduzierung der Inhaltstoffe Ammoniak, Phosphat, Ammonium, Nitrat, Nitrit, Amine, Schwefelwasserstoff, Alkohole, Ketone, Ester, organische Säuren, organische Schwefelverbindungen, aromatische Kohlenwasserstoffe und sonstige Kohlenwasserstoffverbindungen und/oder Kohlendioxid in der Abluft und im Prozesswasser einer Anlage, mit den Schritten,
a) das Prozesswasser und die Abluft durch eine Wäschereinheit zu leiten, wobei das Prozesswasser die Inhaltstoffe aus der Abluft aufnimmt,
b) das mit den Inhaltstoffen angereicherte Prozesswasser zumindest teilweise in eine Mikroalgen enthaltende Konversionsvorrichtung einzuleiten, in welcher die Inhaltstoffe zumindest teilweise von den Mikroalgen aufgrund von photosynthetischer Aktivierung aufgenommen werden, und
c) die Mikroalgen nach Aufnahme der Inhaltstoffe zumindest teilweise vom Prozesswasser zu trennen.

Ferner wird gemäß einem zweiten Aspekt der Erfindung vorgeschlagen eine Vorrichtung zur Reduzierung der Inhaltstoffe Ammoniak, Phosphat, Ammonium, Nitrat, Nitrit, Amine, Schwefelwasserstoff, Alkohole, Ketone, Ester, organische Säuren, organische Schwefelverbindungen, aromatische Kohlenwasserstoffe und sonstige Kohlenwasserstoffverbindungen und/oder Kohlendioxid in der Abluft und im Prozesswasser einer Anlage, mit einer Wäschereinheit zur Aufnahme der Inhaltstoffe aus der Abluft durch das Prozesswasser, wobei das Prozesswasser und die Abluft durch die Wäschereinheit leitbar und in der Wäschereinheit miteinander in Berührung bringbar sind, einer mit der Wäschereinheit verbundenen, Mikroalgen enthaltenden Konversionsvorrichtung zur zumindest teilweisen photosynthetischen Aufnahme von Inhaltstoffen aus dem mit Inhaltstoffen angereicherten Prozesswasser durch die Mikroalgen und einem die Wäschereinheit umfassenden, ersten, Kreislauf für das Prozesswasser, in welchem dei Abluft mit dem Prozesswasser in Kontakt kommt, und einem zumindest einen Teil der Wäschereinheit und die Konversionsvorrichtung umfassenden, zweiten Kreislauf für das Prozesswasser, in welchem die Mikroalgen Inhaltsstoffe aus dem Prozesswasser aufnehmen, wobei der erste Kreislauf die Wäschereinheit, einen in die Wäschereinheit führenden ersten Einlaß und mit einem Durchmischungsabschnitt der Wäschereinheit verbundenen ersten Auslaß sowie eine den ersten Einlaß und den ersten Auslaß verbindende erste Verbindungsleitung umfaßt, und der zweite Kreislauf den Durchmischungsabschnitt der Wäschereinheit, einen mit diesem Durchmischungsabschnitt verbundenen zweiten Einlaß und einen zweiten Auslaß, die Konversionsvorrichtung sowie eine zweite Verbindungsleitung zwischen dem zweiten Auslaß und der Konversionsvorrichtung und eine dritte Verbindungsleitung zwischen der Konversionsvorrichtung und dem zweiten Einlaß umfaßt, und einer der Konversionsvorrichtung nachgeschalteten Separationseinrichtung zum zumindest teilweise Trennen der Mikroalgen vom Prozesswasser.

Die Vorteile der Erfindung liegen insbesondere darin, dass die Mikroalgen das Prozesswasser reinigen, nachdem das Prozesswasser Inhaltsstoffe aus der Abluft - eventuell in umgewandelter, beispielsweise mineralisierter, Form - aufgenommen hat. Die Mikroalgen nehmen hierbei in der Konversionsvorrichtung die eventuell umgewandelten Inhaltsstoffe unter Kohlendioxidverbrauch und Sauerstoffproduktion auf bzw. bauen diese ein, wodurch sie sich vermehren. Anschließend werden die Mikroalgen zumindest teilweise vom Prozesswasser getrennt und können zur weiteren wirtschaftlichen Verwertung, beispielsweise zur Schwermetallsorption aus Abwässern, eingesetzt werden. Das gereinigte Prozesswasser hingegen kann z.B. zurück in eine landwirtschaftliche oder industrielle Anlage geführt oder im Prinzip beliebig oft in die Wäschereinheit zurückgeleitet werden. Eine aufwändige und die Umwelt belastende Entsorgung des Prozesswassers entfällt. Als Endprodukte entstehen somit gereinigte Abluft, sauberes Prozesswasser sowie Mikroalgenbiomasse, die weiter verwertet werden können.

Generell kann Abluft unterschiedlichster Art wie beispielsweise kohlendioxidhaltige Abluft in der Wäschereinheit von ihren Inhaltsstoffen separiert werden. Voraussetzung ist lediglich, dass die Inhaltsstoffe biologisch bindbar sind.

Demnach wird gemäß der Erfindung als Gas Abluft einer landwirtschaftlichen oder industriellen Anlage sowie als Flüssigkeit Prozesswasser verwendet, welches Inhaltsstoffe in Form von Nährstoffen für die Mikroalgen enthält und beispielsweise aus derselben landwirtschaftlichen oder industriellen Anlage stammen kann. Konkret wird beispielsweise kohlendioxidreiche Verbrennungsluft sowie gleichzeitig entstehendes Prozesswasser einer Biogasverbrennungsanlage der Wäschereinheit zugeführt. In einer industriellen Anwendung sind z.B. die anfallende Abluft sowie das Prozesswasser zusammen mittels des erfindungsgemäßen Verfahrens und der erfindungsgemäßen Vorrichtung reinigbar.

Nachdem die Mikroalgen in der Konversionsvorrichtung Inhaltsstoffe aus der Flüssigkeit aufgenommen haben, wird die Flüssigkeit - ggf. vor Abtrennung der Mikroalgen - zumindest teilweise in die Wäschereinheit zurückgeleitet. Hierdurch lässt sich mit Vorteil ein kontinuierlicher Durchlaufbetrieb der Flüssigkeit realisieren. Die in der Konversionsvorrichtung gereinigte Flüssigkeit wird dann in der Wäschereinheit von Neuem mit dem verunreinigten Gas in Berührung gebracht und anschließend wieder zur Konversionsvorrichtung geleitet.

Demnach läßt sich das erfindungsgemäße Verfahren prinzipiell mit einem einzigen Kreislauf für die Flüssigkeit durchführen, bei dem die Flüssigkeit von einem Auslaß der Wäschereinheit zur Konversionsvorrichtung und anschließend zu einem Einlaß der Wäschereinheit zurückgeleitet wird, Hierdurch wird vorteilhafterweise ein relativ einfacher Aufbau der Vorrichtung zur Durchführung des erfindungsgemäßen Verfahrens realisiert.

Andererseits hat es sich als vorteilhaft erwiesen, wenn die Flüssigkeit in zwei Teilströme aufgeteilt wird, die in zwei Kreisläufen - mit ggf. beiden Kreisläufen gemeinsamen Teilabschnitten - zirkulieren. Vorzugsweise wird ein Flüssigkeitsteilstrom in einem ersten Kreislauf von einem ersten Auslaß der Wäschereinheit zu einem ersten Einlaß der Wäschereinheit zurückgeleitet, während ein anderer Flüssigkeitsteilstrom in einem zweiten Kreislauf von einem zweiten Auslaß der Wäschereinheit zur Konversionsvorrichtung geleitet und nach Durchlaufen der Konversionsvorrichtung zu einem zweiten Einlaß der Wäschereinheit zurückgeleitet wird.

Vorzugsweise durchmischen sich die Flüssigkeitsteilströme der beiden Kreisläufe zumindest in einem Durchmischungsabschnitt der Wäschereinheit. Hierzu hat es sich als vorteilhaft gezeigt, wenn sich die Flüssigkeitsteilströme in einem als Reservoir dienenden Durchmischungsabschnitt der Wäschereinheit sammeln und sich dort durchmischen. Das Reservoir stellt hierbei einen beiden Kreisläufen gemeinsamen Teilabschnitt dar. Vom Reservoir aus wird dann die Flüssigkeit einerseits durch den ersten Auslaß über den ersten Kreislauf zurück zur Wäschereinheit und andererseits durch den zweiten Auslaß über den zweiten Kreislauf zur Konversionsvorrichtung geleitet. Hierdurch wird gewährleistet, daß die in beiden Kreisläufen zirkulierende Flüssigkeitsteilströme im wesentlichen dieselben Inhaltsstoffe enthalten, wodurch die der Wäschereinheit über den ersten Kreislauf zugeführte Flüssigkeit kontinuierlich mit der von der Konversionsvorrichtung kommenden gereinigten Flüssigkeit 'verdünnt' ist.

Zur konstruktiven Vereinfachung der Vorrichtung können der erste und zweite Auslaß vorzugsweise einen gemeinsamen Auslaß bilden.

Eine bevorzugte Ausführungsform zeichnet sich dadurch aus, daß vom gemeinsamen Auslaß der Wäschereinheit eine Leitung abgeht, die sich im weiteren Verlauf in eine erste Verbindungsleitung und in eine zweite Verbindungsleitung gabelt. Die zum ersten Kreislauf gehörende erste Verbindungsleitung leitet einen Flüssigkeits-teilstrom zur Wäschereinheit zurück, während die zum zweiten Kreislauf gehörende zweite Verbindungsleitung einen Flüssigkeitsteilstrom zur Konversionsvorrichtung führt.

Bevorzugt ist die Wäschereinheit als Rieselbettreaktor ausgebildet, in den Füllkörper aus Kieselsteinen, Lavamaterial, Kunststoffen oder anderem Material mit großer Oberfläche gefüllt sind. Die Flüssigkeit - beispielsweise über den ersten Kreislauf herantransportiert - berieselt die Füllkörper, wobei die in der Flüssigkeit stets vorhandenen Mikroalgen sich auf der Oberfläche der Füllkörper ablagern und dort vermehren. Enthält die Flüssigkeit eine vermehrte Anzahl an Bakterien, können sich diese in entsprechender Zahl auf den Füllkörpern absetzen. Bei der Berieselung der Füllkörper benetzt die Flüssigkeit deren Oberfläche, so daß die Moleküle der Inhaltsstoffe aus dem Gas an dem Flüssigkeitsfilm sowie den Mikroalgen auf den Füllkörpern adsorptiv gebunden werden und von den Mikroalgen unter Aufnahme von Sauerstoff und Abgabe von Kohlendioxid mineralisiert werden. Auf diese Weise reichert sich die Flüssigkeit mit mineralisierten Inhaltsstoffen (auch Nährstoffe genannt) an, wozu z.B. Phosphate, Nitrate, Ammonium usw, zählen.

Der Sauerstoff für die auf den Füllkörpern abgelagerten Mikroalgen wird vorteilhafterweise von der Flüssigkeit herantransportiert, welche zuvor in der Konversionsvorrichtung aufgrund der Photosyntheseaktivität der Algen mit Sauerstoff angereichert wurde und über das Reservoir und den ersten Kreislauf zu den Füllkörpern gelangt. Teilweise reichert der Sauerstoff in der Flüssigkeit das die Wäschereinheit verlassende, gereinigte Gas an.

In einer vorteilhaften Ausführungsform der Erfindung ist vorgesehen, daß durch entsprechende Zufuhr zum einzigen Kreislauf bzw. zum ersten und/oder zweiten Kreislauf die Menge an Inhaltsstoffen beispielsweise in Form von mineralisierten Nährstoffen und/oder die Algenmenge erhöht werden können. Hierdurch sind die einzelnen Mengen - evtl. mit Hilfe einer geeigneten Meß- und Regelungseinrichtung - derart aufeinander abstimmbar, daß eine optimierte Reinigung von Gas und Flüssigkeit erreicht werden kann. Hierbei ist zu beachten, daß eine hohe Algenproduktion erwünscht ist, da bei hohem Algenanteil auch die Nährstoffaufnahme, der Kohlendioxidverbrauch sowie die Sauerstoffproduktion seitens der Algen zunimmt. Wird jedoch beispielsweise ein Grenzwert der optischen Dichte überschritten, z.B. bei zu großer Algendichte, kann verdünnende Nährlösung mit mineralisierten Inhaltsstoffen oder auch frisches Wasser aus einem Vorratsbehälter oder einer Zulaufeinrichtung dem ersten und/- oder dem zweiten Kreislauf zugeführt werden, Alternativ oder zusätzlich kann zur Herabsetzung der optischen Dichte ein Teil der Algen vor Rückführung der Flüssigkeit zu den Kreisläufen abgetrennt werden.

Eine Algenpopulation kann sich unter günstigen Bedingungen selbst einstellen; es können jedoch auch Algen, beispielsweise ein bestimmter Algenstamm, inokuliert werden. Diese Inokulation wird entweder vor oder während des Betriebs der erfindungsgemäßen Vorrichtung vorgenommen. Beispielsweise kann eine Zuführleitung vorgesehen sein, welche Algen kontinuierlich oder je nach Bedarf in einen der Kreisläufe nachführt.

Zwar findet der Stoffübertrag vom Gas auf die Flüssigkeit mit Hilfe der Mikroalgen vornehmlich in der Wäschereinheit statt, während der Stoffübertrag von der Flüssigkeit auf die Mikroalgen vornehmlich in der Konversionsvorrichtung abläuft, jedoch kann zweckmäßigerweise die umlaufende Flüssigkeit sowohl Mikroalgen, die hauptsächlich in der Wäschereinheit aktiv sind, als auch Mikroalgen enthalten, welche im wesentlichen in der Konversionsvorrichtung nötig sind.

Die Wäschereinheit kann nach dem Gleichstromprinzip oder dem Gegenstromprinzip ausgelegt sein, d.h. das Gas und die Flüssigkeit durchlaufen die Wäschereinheit im wesentlichen in gleicher oder entgegengesetzter Richtung. Je nach besserer, vom Einzelfall abhängigen Stoffübertragung wird das eine oder andere Prinzip eingesetzt. Bei Verwendung eines Gegenstrom-Sprühwäscher sind ggf. auch keine Füllkörper in der Wäschereinheit vorgesehen.

Die Konversionsvorrichtung weistbevorzugt mindestens einen Photobioreaktor auf, in welchem die Algen durch Bestrahlung mittels Sonnenlicht und/oder künstlichem Licht die z.B. von den Mikroalgen mineralisierten Inhaltsstoffe aus der Flüssigkeit unter Erzeugung von Sauerstoff und Aufnahme von Kohlendioxid aufnehmen bzw. einbauen. Photobioreaktoren haben den Vorteil, daß sie wartungsfreundlich sind und nur einen geringen Platz benötigen, da sie üblicherweise auf einer relativ kleinen Grundfläche aufstellbar sind. Bei bekannten Photobioreaktoren wird die zu bestrahlende Flüssigkeit durch ein mehrfach gewundenes, transparentes Rohr geleitet, in welches das Licht eindringen kann, um die in der Flüssigkeit enthaltenen Mikroalgen zur photosynthetischen Aufnahme der Inhaltsstoffe aus der Flüssigkeit anzuregen.

Als Konversionsvorrichtung sind generell halb-offene Photobioreaktoren einsetzbar. Beispielsweise ist anstelle eines kegelstumpfförmig ausgebildeten Photobioreaktors auch ein Plattenphotobioreaktor verwendbar. Ebenfalls kann ein Fermenter mit Innenbeleuchtung eingesetzt werden.

Alternativ oder ggf. auch zusätzlich kann die Konversionsvorrichtung ein offenes Algenkultivationssystem aufweisen.

Da - bei einer zwei Kreisläufe aufweisenden Vorrichtung - die Mikroalgen mit der Flüssigkeit vor ihrer Abtrennung in den beiden Kreisläufen zirkulieren, muß verhindert werden, daß die Mikroalgen den ersten und zweiten, aus der Wäschereinheit herausführenden Auslaß verstopfen. Hierzu wird die Flüssigkeit bevorzugt durch den zweiten Einlaß unter Wirbelbildung derart in das Reservoir der Wäschereinheit zurückgeleitet, daß die Flüssigkeit sich ständig in einer Wirbelbewegung befindet und somit keine Mikroalgen den ersten und zweiten bzw. den gemeinsamen Auslaß blockieren.

Zur Abtrennung der Mikroalgen von der Flüssigkeit hat es sich als vorteilhaft erwiesen, diese dann aus der Wäschereinheit abzuleiten, wenn die Flüssigkeit in dem Reservoir einen vorgegebenen Füllstand im Reservoir übersteigt. Der Grund für die mit der Zeit ansteigende Flüssigkeitsmenge in dem Reservoir liegt insbesondere darin, daß - wie vorerwähnt - aus einem Vorratsbehälter oder einer Zulaufeinrichtung Flüssigkeit, beispielsweise mit mineralisierten Inhaltsstoffen angereichertes Prozeßwasser, dem ersten und/oder zweiten Kreislauf zugeführt wird. Der Zufluß kann in Abhängigkeit von der Algendichte im Konversionssystem geregelt werden, oder es wird eine konstante Zuflußrate eingestellt. Das überschüssiger Wasser verläßt die Wäschereinheit zweckmäßigerweise durch einen dritten Auslaß mittels einer Überlaufeinrichtung und wird in eine Separationseinrichtung geleitet, die beispielsweise eine Zentrifuge und/oder eine Eindickvorrichtung zurAbtrennung der Mikroalgen von der Flüssigkeit aufweist. Die abgetrennte Algenbiomasse kann dann einer weiteren Verwertung zugeführt werden.

Um Ablagerungen von Mikroalgen an den Rohrinnenwänden des zweiten Kreislaufes zu vermeiden, welche insbesondere die Transparenz der Rohre des oder der Photobioreaktoren und damit den Nährstoffeinbau seitens der Algen beeinträchtigen würden, ist dem zweiten Kreislauf ein Reinigungsgranulat zugesetzt, welches aufgrund der Strömung der Flüssigkeit an den Rohrinnenwänden scheuert und dort angelagerte Mikrobioalgen mitnimmt. Damitdas Reinigungsgranulat jedoch nicht zu der Berieselungsvorrichtung sowie den Füllkörpern gelangt, sind Filter im ersten Kreislauf vorgesehen, welche das Reinigungsgranulat weitestgehend abhalten. Vorzugsweise ist mindestens ein Filter, welcher beispielsweise als Sieb ausgebildet sein kann, vor dem ersten Auslaß - falls erster und zweiter Auslaß getrennt sind - im Reservoir angeordnet. Falls ein gemeinsamer Auslaß vorgesehen ist, ist der Filter vorzugsweise derart im Bereich der Abzweigung von erstem und zweitem Kreislauf angeordnet, daß vom Reservoir in den zweiten Kreislauf fließendes Wasser am Filter abgelagertes Reinigungsgranulat mitreißt und dieser nicht verstopft.

Vorteilhafterweise ist in dem einzigen Kreislauf bzw. im ersten Kreislauf eine erste steuerbare Pumpenvorrichtung für eine bedarfsgerechte Benetzung der Füllkörper mit der Flüssigkeit vorgesehen. Je nach momentan in die Wäschereinheit eingebrachtem Gas sowie deren Verschmutzungsgrad sorgt die erste Pumpenvorrichtung für eine optimale Berieselung der Füllkörper.

Eine zweite Pumpenvorrichtung wälzt die Flüssigkeit im zweiten Kreislauf bevorzugt kontinuierlich um. Hierdurch wird ein fortwährender Eintrag der Flüssigkeit in das Reservoir zur Freihaltung des ersten und zweiten bzw. des gemeinsamer Auslasses - wie oben beschrieben - gewährleistet. Außerdem ist die Wahrscheinlichkeit einer Verstopfung der Auslässe aufgrund der Sogwirkung der zweiten Pumpenvorrichtung gering. Zudem werden durch den kontinuierlichen Durchlaufbetrieb die Rohre der Photobioreaktoren mittels des Reinigungsgranulats permanent freigehalten.

Eine Meß- und Regelungseinrichtung führt bevorzugt in zeitlichen Abständen oder kontinuierlich Messungen von Parametern der Flüssigkeit, wie pH-Wert, optischer Dichte, Sauerstoffkonzentration, elektrischer Leitfähigkeit, Temperatur, Volumenteilströme, durch. Auf Grundlage dieser Meßwerte sind einzelne Verfahrensschritte regelbar. Die Datenerfassung und -visualisierung ist vorteilhafterweise über Datenfernüberwachung und Datenfernabfrage steuerbar, was den Personalaufwand vor Ort reduziert. Der pH-Wert der Flüssigkeit sollte sich mittels einer externen Kohlendioxidquelle regulieren lassen. Hierzu wird der von der Meß- und Regelungseinrichtung gemessene pH-Wert der Flüssigkeit ausgewertet und bei Bedarf die Kohlendioxidquelle zur Zufuhr von Kohlendioxid aktiviert.

Vorteilhafte Weiterbildungen der Erfindung sind im übrigen durch die Merkmale der Unteransprüche gekennzeichnet.

Im folgenden wird die Erfindung anhand der Zeichnungen näher erläutert. Es zeigen:
- Fig. 1: eine schematische Darstellung einer Wäschereinheit, einer aus drei Photobioreaktoren bestehenden Konversionsvorrichtung sowie einer Separationseinrichtung gemäß einer ersten Ausführungsform;
- Fig. 2: eine vergrößert Darstellung der Wäschereinheit und der Separationseinrichtung gemäß der Fig. 1;
- Fig. 3: eine schematische Darstellung einer zweiten Ausführungsform; und
- Fig. 4: eine schematische Darstellung einer dritten Ausführungsform.

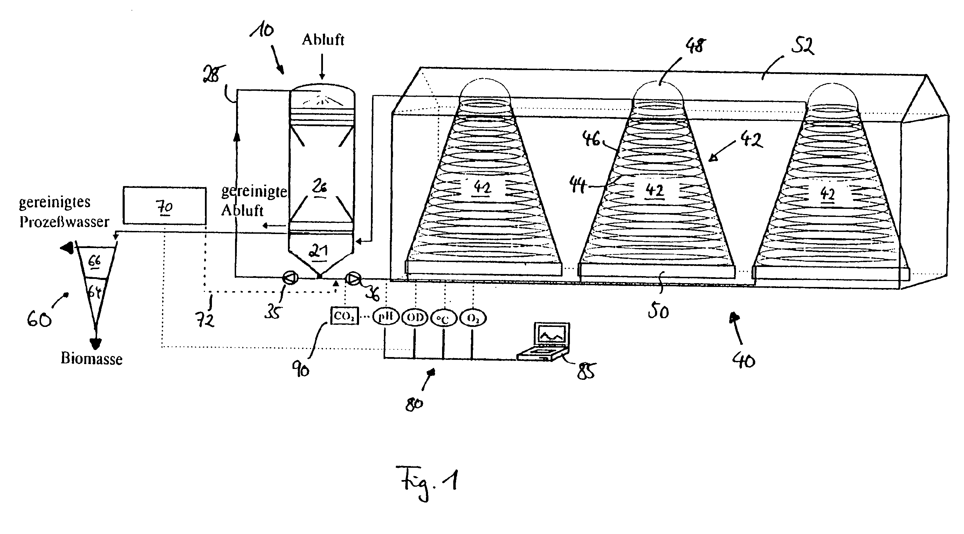

Fig. 1 zeigt eine schematische Gesamtansicht einer ersten Ausführungsform der erfindungsgemäßen Vorrichtung, deren wesentliche Bestandteile eine Wäschereinheit 10, eine Konversionsvorrichtung 40 sowie eine Separationseinrichtung 60 sind.

In der Wäschereinheit 10 kommt mit Inhaltsstoffen beladene Abluft mit Prozesswasser in Berührung, wodurch das Prozesswasser Inhaltsstoffe aus der Abluft ggf. nach Umwandlung der Inhaltsstoffe, wie weiter unten ausgeführt - aufnimmt. Während die gereinigte Abluft die Wäschereinheit 10 durch einen separaten Gasauslaß 24 verläßt, wird die Flüssigkeit zum Teil in einem ersten Kreislauf über eine erste Verbindungsleitung 28 wieder in die Wäschereinheit 10 zurückgeleitet, während ein anderer Teil der Flüssigkeit in einem zweiten Kreislauf über eine zweite Verbindungsleitung 29 zur Konversionsvorrichtung 40 geleitet wird, welche aus drei parallel geschalteten Photobioreaktoren 42 besteht. Die im zweiten Kreislauf zirkulierende Flüssigkeit enthält Mikroalgen, die unter Lichtbestrahlung in dem Photobioreaktor 42 Inhaltsstoffe aus der Flüssigkeit einbaut. Die so an Inhaltsstoffen reduzierte Flüssigkeit sowie die in der Flüssigkeit schwimmenden Mikroalgen werden von der Konversionsvorrichtung 40 über eine dritte Verbindungsleitung zur Wäschereinheit 10 zurückgeleitet und von dort zum Teil in die Separationseinrichtung 60 geführt, in der die Mikroalgen von der Flüssigkeit getrennt werden.

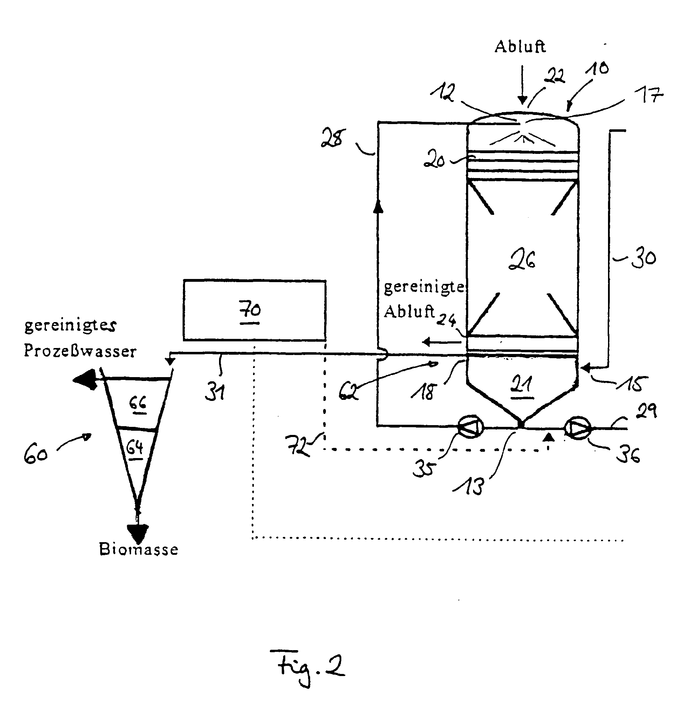

In Fig. 2 ist die Anordnung von Wäschereinheit 10 und Separationseinrichtung 60 der Fig. 1 im einzelnen dargestellt. In der ersten Ausführungsform gemäß der Fig. 1 ist die Wäschereinheit 10 als im wesentlichen zylindrischer Rieselbettreaktor 10 ausgebildet. An der Oberseite des Rieselbettreaktors 10 ist zentral ein Gaseinlaß 22 vorgesehen, in dessen Nähe seitlich ein erster Einlaß 12 für das Prozesswasser angeordnet ist, welcher in eine unterhalb des Gaseinlasses 22 angeordnete Sprüheinrichtung 17 mündet, die nach unten hin offen und auf drei untereinander liegende Lagen von Röhrensieben 20 gerichtet ist. Unterhalb der Röhrensiebe 20 schließt sich ein Abschnitt an, in den Füllkörper 26 mit einer großen Oberfläche, wie beispielsweise Kunststoffe, Kieselsteine, Lava, gefüllt sind. Auf der Oberfläche der Füllkörper 26 lagern sich vorzugsweise heterotrophe Mikroalgen ab, die sich dort vermehren können.

In einem unteren Abschnitt des Rieselbettreaktors 10, unterhalb der Füllkörper 26, schließt sich ein nach unten trichterförmig zulaufendes Reservoir 21 an, in dem sich die Flüssigkeit sammelt und in der sich die Flüssigkeitsteilströme des ersten und zweiten Kreislaufes durchmischen. In geringem Abstand oberhalb der Flüssigkeitsoberfläche ist der Gasauslaß 24 in einer Seitenwand des Rieseibettreaktors 10 vorgesehen, durch den das Gas den Rieselbettreaktor 10 nach Reinigung verläßt. An dem spitz zulaufenden unteren Ende des Reservoirs 21 ist ein Auslaß 13 für die Flüssigkeit vorgesehen, der einerseits über die erste Verbindungsleitung 28 mit dem ersten Einlaß 12 und andererseits über die zweite Verbindungsleitung 29 mit der Konversionsvorrichtung 40 verbunden ist. Die von der Konversionsvorrichtung 40 fortführende dritte Verbindungsleitung 30 geht in einen zweiten Einlaß 15 des Rieselbettreaktors 10 über und mündet im wesentlichen tangential, also gegenüber der radialen Richtung versetzt, in das im Querschnitt im wesentlichen kreisförmige Reservoir 21 ungefähr auf Höhe des Flüssigkeitfüllstandes.

In der ersten und der zweiten Verbindungsleitung 28, 29 ist jeweils eine Pumpenvorrichtung 35, 36 vorgesehen, die bedarfsabhängig die Flüssigkeitsteilströme im ersten oder zweiten Kreislauf fördern. Beilspielsweise wird die Flüssigkeit im ersten Kreislauf derart mittels der ersten, steuerbaren Pumpenvorrichtung 35 gepumpt, daß die Füllkörper 26 in Abhängigkeit von der in den Rieselbettreaktor 10 eingetragenen Gasmenge sowie von deren Gehalt an Inhaltsstoffen berieselt werden, während die Flüssigkeit mitsamt der Mikroalgen im zweiten Kreislauf z.B. kontinuierlich zwischen dem Reservoir 21 und der Konversionsvorrichtung 40 umgewälzt wird.

Jeder der drei Photobioreaktoren 42 der Konversionsvorrichtung 40 weist ein aufrecht auf einem Sockel 50 befestigtes kegelstumpfförmiges Trägergestell 46 auf, auf dessen Außenseite ein transparentes Rohr 44 aufgewickelt ist (Fig. 1). Die zweite Verbindungsleitung 29 ist mit dem einen, im Bereich des Sockels 50 liegenden Ende jeden Rohrs 44 verbunden, während die dritte Verbindungsleitung 30 an das andere Ende jeden Rohrs 44 im oberen Abschnitt des Photobioreaktors 42 angeflanscht ist. Die Flüssigkeit tritt in jeden Photobioreaktor 42 im Bereich des Sockels 50 ein und wird innerhalb des durchgehenden Rohrs 44 in Windungen in einen oberen Bereich geleitet, von dem aus sie zum zweiten Einlaß 15 geführt wird. Alternativ ist die Flüssigkeit natürlich auch in entgegengesetzter Richtung durch die Photobioreaktoren leitbar.

Die Photobioreaktoren 42 sind derart aufgestellt, daß Sonnenlicht und gegebenenfalls auch eine im Reaktorinnenraum angeordnete künstliche Lichtquelle die unter Photosynthese erfolgende Aufnahme der mineralisierten Inhaltsstoffe aus der Flüssigkeit durch die Algen hervorrufen. In der dargestellten Ausführungsform sind die drei Photobioreaktoren 42 in einem speziellen ortsfesten Container 52 aufgestellt. Alternativ können die Photobioreaktoren 42 in einem verfahrbaren Container angeordnet sein, um flexible Einsatzmöglichkeiten zu ermöglichen.

Nach oben schließt jeweils eine als Haube ausgebildete, plankonvexe Sammellinse 48 den zugehörigen Photobioreaktor 42 ab, welche einerseits den Reaktorinnenraum bei ungünstigen Witterungen schützt und andererseits zu große Temperaturschwankungen im Reaktor 42 unterbindet. Die Sammellinse 48 bündelt das einfallende Sonnenlicht und erhöht somit die Photosyntheseaktivität der Mikroalgen, Gegebenenfalls ist der Sammellinse 48 gegenüber im Bereich des Sockels 50 ein (nicht dargestellter) Innenreflektor mit einer spiegelnden Oberfläche angeordnet, welcher das Licht auf die Rohre 44 reflektiert und somit die Nährstoffaufnahme seitens der Algen erhöht.

Dem zweiten Kreislauf, der das Reservoir 21, die zweite Verbindungsleitung 29, die Konversionsvorrichtung 40 sowie die dritte Verbindungsleitung 30 umfaßt, ist ein (nicht dargestelltes) Reinigungsgranulat zugesetzt, welches aufgrund der Flüssigkeitsströmung an den Innenwänden der Verbindungsleitungen 29, 30 sowie den Rohren 44 der Photobioreaktoren 42 scheuert und somit verhindert, daß sich dort Mikroalgen absetzen.

Um das relativ großvolumige Reinigungsgranulat weitgehend von der Sprüheinrichtung 17 und den Röhrensieben 20 fernzuhalten, ist ein (nicht dargestellter) Filter stromabwärts des gemeinsamen Auslasses 13 an der Einmündung in die erste Verbindungsleitung 28 vorgesehen. Das Reinigungsgranulat wird von der in den zweiten Kreislauf strömenden Flüssigkeit mitgenommen und sorgt für eine Selbstreinigung des Filters. Falls kein Filter vorhanden wäre, bestünde die Gefahr, daß das Reinigungsgranulat zum Einlaß 12 des Rieselbettreaktors 10 transportiert wird und die Röhrensiebe 20 verstopft bzw, die Wirksamkeit der Füllkörper 26 beeinträchtigt.

Im oberen Abschnitt des Reservoirs 21 ist eine Überlaufeinrichtung mit einem dritten Auslaß 18 für die Flüssigkeit vorgesehen, der über eine vierte Verbindungsleitung 31 mit der Separationsvorrichtung 60 verbunden ist. Erreicht die Flüssigkeit in dem Reservoir 21 einen vorgegebenen Füllstand, fließt die Flüssigkeit zur Separationsvorrichtung 60, welche beispielsweise Durchlaufzentrifugen und/oder Filtereinrichtungen aufweist. In der Separationsvorrichtung 60 werden die Mikroalgen von der Flüssigkeit getrennt, indem die Mikroaigen sich beispielsweise in einem unteren Abschnitt 64 der Separationsvorrichtung 60 absetzen und dort entnommen werden können, während die von Mikroalgen gereinigte Flüssigkeit aus einem oberen Abschnitt 66 der Separationsvorrichtung 60 abfließen kann.

Einige Parameter der Flüssigkeit im zweiten Kreislauf werden kontinuierlich oder in Intervallen gemessen. Hierzu ist eine Meß- und Regelungseinrichtung 80 vorgesehen (schematisch in Fig. 1 unterhalb der Konversionsvorrichtung 40 dargestellt), welche beispielsweise den pH-Wert, die optische Dichte, die Temperatur und/oder der Sauerstoff-Gehalt mißt und diese Werte an eine Datenauswertevorrichtung 85 übergibt. Die Daten sind vorzugsweise von einem entfernten Ort abrufbar.

Anhand von Meßwerten bestimmter Parameter sind einige Verfahrensschritte regelbar. Beispielsweise kann der pH-Wert des Prozesswassers über eine externe Kohlendioxid-Quelle 90 reguliert werden. Kohlendioxid wird dann in den zweiten Kreislauf eingeleitet. Weiterhin wird eine mineralisierte Inhaltsstoffe enthaltenden Lösung oder auch frisches Wasser aus einem Vorratsbehälter 70 über eine Zuführleitung 72 dem zweiten Kreislauf zugeführt, wenn die Meß- und Regelungseinrichtung 80 das Überschreiten eines vorgegebenen Grenzwertes der optischen Dichte der zirkulierenden Flüssigkeit mit den darin enthaltenen Mikroalgen meldet. Dies ist beispielsweise dann der Fall, wenn die Algen einen Großteil der Inhaltsstoffe aus der Flüssigkeit aufgenommen und sich vermehrt haben und deshalb neue Nährlösung zugeführt werden muß, um einerseits neue Nährstoffe zur Verfügung zu stellen und andererseits die in den Kreisläufen zirkulierende Flüssigkeit zu verdünnen. Alternativ zu einer von der Algendichte abhängigen Zuführung der Lösung kann auch ein konstanter Zufluß eingestellt sein.

Nachstehend wird der Betrieb der in den Fig. 1 und 2 dargestellten Ausführungsform der Erfindung näher erläutert. Als Gas wird Abluft verwendet, welche in landwirtschaftlichen oder industriellen Anlagen anfällt, beispielsweise in einer Kompostieranlage, und entsprechend mit Inhaltsstoffen beladen ist, Als Flüssigkeit findet Prozeßwasser Verwendung, welches ggf. in derselben Anlage oder einer sonstigen Anlage des Betriebs eingesetzt wird. Die Abluft wird durch den Gaseinlaß 22 in den Rieselbettreaktor 10 geleitet. Gleichzeitig gelangt Prozeßwasser durch den ersten Einlaß 12 in den Rieselbettreaktor 10 und wird von oben auf die Röhrensiebe 20 gesprüht. Der Rieselbettreaktor 10 arbeitet demnach im Gleichstromprinzip, d.h. die Bewegungsrichtungen von Abluft und Prozeßwasser stimmen im wesentlichen überein. Von den Röhrensieben 20 gelangt das Prozeßwasser auf die Füllkörper 26 und benetzt deren Oberfläche, auf der sich in natürlicher Weise Mikroalgen abgelagert haben. Es ist hierbei anzumerken, daß die Mikroalgen nicht nur auf der Füllkörperoberfläche zu finden, sondern auch in dem in zwei Kreisläufen zirkulierende Prozeßwasser enthalten sind.

Die Aufnahme der Inhaltsstoffe aus der Abluft durch das Prozeßwasser kann auf zweierlei Art erfolgen: Einerseits können die Prozeßwassertropfen und die Moleküle der Inhaltsstoffe in der Abluft direkt zusammenprallen, wobei günstigenfalls die Inhaltsstoffe, zu denen beispielsweise Ammoniak und Kohlendioxid zählen, adsorptiv an die Prozeßwassertropfen gebunden werden. Weitere Inhaltsstoffe in der Abluft werden von dem die Füllkörper 26 benetzenden Prozeßwasser gebunden, indem die Moleküle dieser Inhaltsstoffe mit den Mikroalgen auf der Füllkörperoberfläche in Berührung kommen, von diesen unter Verbrauch von Sauerstoff mineralisiert und als anorganische, mineralisierte Inhaltsstoffe, beispielsweise in Form von Phosphaten, Nitraten, Ammonium usw., an das Prozeßwasser abgegeben werden.

Das Prozeßwasser läuft unter Wirkung der Schwerkraft an den Füllkörpern 26 entlang nach unten und sammelt sich in dem trichterförmigen Reservoir 21 des Rieselbettreaktors 10. Von dort wird ein Teil des Prozeßwassers durch den gemeinsamen Auslaß 13 im ersten Kreislauf zum ersten Einlaß 12 zurück und ein anderer Teil im zweiten Kreislauf kontinuierlich zu den Photobioreaktoren 42 gepumpt, in denen das Prozeßwasser mit Sonnenlicht und/oder künstlichem Licht bestrahlt wird. Aufgrund der großen Sogwirkung der zweiten Pumpenvorrichtung 36 ist ein Verstopfen des gemeinsamen Auslasses 13 durch die Mikroalgen weitgehend auszuschließen. Die Mikroalgen, die mit dem Prozeßwasser in dem ersten und zweiten Kreislauf zirkulieren, nehmen die mineralisierten Inhaltsstoffe aus dem Prozeßwasser unter dem Lichteinfluß in den Photobioreaktoren 42 auf. Für diesen Prozeß ist Kohlendioxid notwendig, welches u.a. von den Mikroalgen produziert wird, sich im Prozeßwasser anreichert und von diesem zu den Photobioreaktoren 42 transportiert wird. Das durch den zweiten Einlaß 15 in das Reservoir 21 des Rieselbettreaktors 10 zurückgeleitete Prozeßwasser weist nach Durchlaufen der Photobioreaktoren 42 einen reduzierten Inhaltsstoffanteil und einen erhöhten Sauerstoffgehalt aufgrund der Sauerstoffproduktion seitens der Algen während der Photosynthese auf.

Der sich im Prozeßwasser durch die Photosynthese angereicherte Sauerstoff wird einerseits von den Mikroalgen zur Mineralisierung der Inhaltsstoffe benötigt, andererseits reichert er auch die im Rieselbettreaktor 10 zu reinigende Abluft an.

Das Rückleiten des im zweiten Kreislauf zirkulierenden Prozeßwassers in das im unteren Teil des Rieselbettreaktors 10 angeordnete Reservoir 21 erfüllt zwei Funktionen. Einerseits wird aufgrund des kontinuierlichen Durchflusses im zweiten Kreislauf eine stetige Verwirbelung des Prozeßwassers in dem Reservoir 21 erreicht, indem das Prozeßwasser tangential und unter Druck durch den tangential am im Querschnitt kreisförmigen Reservoir 21 sitzenden zweiten Einlaß 15 in das Reservoir 21 geleitet wird. Andererseits gelangt auf diese Weise kein Reinigungsgranulat in die oberen Abschnitte des Rieselbettreaktors 10.

Ist ein vorgegebener Füllstand des Reservoirs 21 erreicht, fließt das Prozeßwasser aus dem dritten Auslaß 18 der Überlaufeinrichtung 62 in die Separationsvorrichtung 60, in der die mit dem Prozeßwasser enthaltenden Mikroalgen abgetrennt werden. Die Algenbiomasse kann dann beispielsweise einer Biogasgewinnungsanlage zugeleitet werden.

Von einem Vorratsbehälter 70 wird Lösung mit beispielsweise mineralisierten Inhaltsstoffen nachgeführt, wenn die von der Meß- und Regelungseinrichtung 80 gemessene optische Dichte des Prozeßwassers mit den darin enthaltenen Mikroalgen einen Grenzwert übersteigt. Alternativ kann in diesem Fall Prozeßwasser, welches ggf. mit mineralisierten Inhaltsstoffen wie Phosphaten, Ammonium, Nitraten, Nitriten usw. angereichert ist sowie eventuell Bakterien enthält, mittels einer (nicht dargestellten) Zulaufeinrichtung z.B. in den zweiten Kreislauf geleitet werden. Wenn bei dieser Ausführungsform das gereinigte Prozeßwasser in die landwirtschaftliche oder industrielle Anlage zurückgeleitet wird, ergibt sich ein vorteilhafter weitgehend geschlossener Stoffkreislauf für das Prozeßwasser. Auch ein kontinuierlicher Zufluß von eventuell mit Inhaltsstoffen angereichertem Prozeßwasser von einer Anlage und somit gleichfalls ein weitgehend kontinuierlicher Rückfluß von gereinigtem Prozeßwasser zur Anlage ist möglich. Anstelle von Prozeßwasser kann alternativ auch frisches Wasser zur Herabsetzung einer übergroßen Algendichte zugeführt werden.

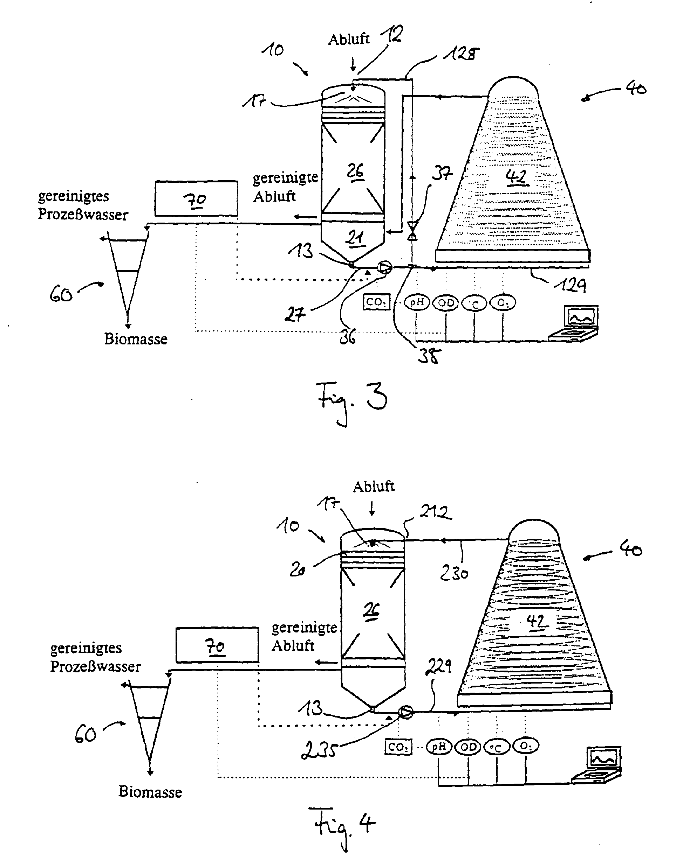

In den Fig. 3 und 4 sind zwei weitere Ausführungsformen der erfindungsgemäßen Vorrichtung dargestellt, die sich von der ersten Ausführungsform im wesentlichen durch die Führung der Flüssigkeit unterscheiden. Die in der Fig. 3 dargestellte Ausführungsform zeichnet sich dadurch aus, daß vom gemeinsamen Auslaß 13 des Rieselbettreaktors 10 eine Leitung 27 abgeht, die sich weiter stromabwärts in eine erste Verbindungsleitung 128 und in eine zweite Verbindungsleitung 129 gabelt. Die erste Verbindungsleitung 128 ist Bestandteil des ersten Kreislaufes für die Flüssigkeit und führt einen Flüssigkeitsteilstrom - kontrolliert mittels eines Ventils 37 - über den ersten Einlaß 12 des Rieselbettreaktors 10 zur Sprüheinrichtung 17. Die zweite Verbindungsleitung 129 ist Teil des zweiten Kreislaufes und führt einen Flüssigkeitsteilstrom zur Konversionsvorrichtung 40. Eine Pumpenvorrichtung 36 in der Leitung 27 fördert die Flüssigkeit kontinuierlich oder intervallartig, während ein Filter 38 im Bereich der Abzweigung der Leitung 27 ein Vordringen von Reinigungsgranulat zur Sprüheinrichtung 17 verhindert.

Im Gegensatz zu den in den Fig. 1 - 3 dargestellten beiden Ausführungsformen weist die dritte, in Fig. 4 abgebildete Ausführungsform lediglich einen einzigen Kreislauf für die Flüssigkeit auf. Hierbei wird die die Konversionsvorrichtung 40 durchlaufende Flüssigkeit mittels einer Pumpenvorrichtung 235 zu einem Einlaß 212 des Rieselbettreaktors 10 und anschließend zur Sprüheinrichtung 17 geleitet. Bei dieser Ausführungsform kann kein Reinigungsgranulat zur Säuberung der Rohre 44 des Photobioreaktions 42 eingesetzt werden, da dieses ansonsten zur Sprüheinrichtung 17 und den Röhrensieben 20 gelangen und diese verstopfen würde.

## Patentansprüche

1. Verfahren zur Reduzierung der Inhaltstoffe Ammoniak, Phosphat, Ammonium, Nitrat, Nitrit, Amine, Schwefelwasserstoff, Alkohole, Ketone, Ester, organische Säuren, organische Schwefelverbindungen, aromatische Kohlenwasserstoffe und sonstige Kohlenwasserstoffverbindungen und/oder Kohlendioxid in der Abluft und im Prozesswasser einer Anlage, mit den Schritten,
a) das Prozesswasser und die Abluft durch eine Wäschereinheit (10) zu leiten, wobei das Prozesswasser die Inhaltstoffe aus der Abluft aufnimmt,
b) das mit den Inhaltstoffen angereicherte Prozesswasser zumindest teilweise in eine Mikroalgen enthaltende Konversionsvorrichtung (40) einzuleiten, in welcher die Inhaltstoffe zumindest teilweise von den Mikroalgen aufgrund von photosynthetischer Aktivierung aufgenommen werden, und
c) die Mikroalgen nach Aufnahme der Inhaltstoffe zumindest teilweise vom Prozesswasser zu trennen.

2. Verfahren nach Anspruch 1,
**dadurch gekennzeichnet, daß** das inhaltsstoffreduzierte Prozesswasser nach Durchlaufen der Konversionsvorrichtung (40) zumindest teilweise in die Wäschereinheit (10) zurückgeführt wird.

3. Verfahren nach Anspruch 1 oder 2,
**dadurch gekennzeichnet, daß** das Prozesswasser in einem einzigen Kreislauf von einem Auslaß (13) der Wäschereinheit (10) zur Konversionsvorrichtung (40) geleitet und nach Durchlaufen der Konversionsvorrichtung (40) zu einem Einlaß (212) der Wäschereinheit (10) zurückgeleitet wird.

4. Verfahren nach Anspruch 1 oder 2,
**dadurch gekennzeichnet, daß** Prozesswasser in einem ersten Kreislauf von einem ersten Auslaß (13) der Wäschereinheit (10) zu einem ersten Einlaß (12) der Wäschereinheit (1 0) zurückgeleitet wird, Prozesswasser in einem zweiten Kreislauf von einem zweiten Auslaß (13) der Wäschereinheit (10) zur Konversionsvorrichtung (40) geleitet und nach Durchlaufen der Konversionsvorrichtung (40) zu einem zweiten Einlaß (15) der Wäschereinheit (10) zurückgeleitet wird, und sich die Flüssigkeitsmengen der beiden Kreisläufe zumindest in einem Durchmischungsabschnitt (21) der Wäschereinheit (10) durchmischen.

5. Verfahren nach Anspruch 4,
**dadurch gekennzeichnet, daß** sich das Prozesswasser nach Anreicherung mit Inhaltsstoffen aus dem Gas zumindest teilweise in einem als Reservoir (21) dienenden Durchmischungsabschnitt der Wäschereinheit (10) sammelt, in welchem sich die Flüssigkeitsmengen des ersten und zweiten Kreislaufes durchmischen, und Prozesswasser aus dem Reservoir (21) durch den ersten Auslaß (13) in den ersten Kreislauf und durch den zweiten Auslaß (13) in den zweiten Kreislauf geleitet wird.

6. Verfahren nach Anspruch 4 oder 5,
**dadurch gekennzeichnet, daß** der erste Auslaß (13) und der zweite Auslaß (13) einen gemeinsamen Auslaß (13) bilden.

7. Verfahren nach Anspruch 6,
**dadurch gekennzeichnet, daß** von einem gemeinsamen Auslaß (13) der Wäschereinheit (10) eine Leitung (27) abgeht, welche sich in eine erste Verbindungsleitung (128) des ersten Kreislaufes zur Zuführung eines Flüssigkeitsteilstromes zu einem ersten Einlaß (12) der Wäschereinheit (10) und in eine zweite Verbindungsleitung (129) des zweiten Kreislaufes zur Zuführung eines Flüssigkeitsteilstromes zur Konversionsvorrichtung (40) gabelt.

8. Verfahren nach einem der Ansprüche 4 bis 7,
**dadurch gekennzeichnet, daß** das Prozesswasser von der Konversionsvorrichtung (40) durch den zweiten Einlaß (15) unter Wirbelbildung derart in das Reservoir (21) der Wäschereinheit (10) zurückgeleitet wird, daß ein Verstopfen der Auslässe (13) durch die Mikroalgen in der Flüssigkeit verhindert wird.

9. Verfahren nach einem der Ansprüche 4 bis 8,
**dadurch gekennzeichnet, daß** überschüssiges Prozesswasser im Durchmischungsabschnitt (21) durch einen dritten Auslaß (18) in der Wäschereinheit (10), vorzugsweise mittels einer Überlaufeinrichtung (62), in eine Separationseinrichtung (60) abgeführt wird, in der die Mikroalgen vom Prozesswasser getrennt werden.

10. Verfahren nach einem der Ansprüche 3 bis 9,
**dadurch gekennzeichnet, daß** die von den Mikroalgen aufnehmbare Inhaltsstoffmenge und/oder die Mikroalgenmenge in dem einzigen Kreislauf bzw. in dem ersten und/oder zweiten Kreislauf durch externe Zufuhr erhöht wird.

11. Verfahren nach einem der Ansprüche 3 bis 10.
**dadurch gekennzeichnet, daß** Flüssigkeit mit von den Mikroalgen aufnehmbaren Inhaltsstoffen aus einem Vorratsbeliälter (70) oder einer Zulaufeinrichtung dem einzigen Kreislauf bzw. dem ersten und/oder dem zweiten Kreislauf zugeführt wird.

12. Verfahren nach einem der Ansprüche 3 bis 11,
**dadurch gekennzeichnet, daß** das in dem einzigen Kreislauf bzw. in beiden Kreisläufen umlaufende Prozesswasser Mikroalgen enthält.

13. Verfahren nach einem der vorhergehenden Ansprüche,
**dadurch gekennzeichnet, daß** das Prozesswasser und/oder die Abluft durch die als Rieselbettreaktor (10) ausgebildete Wäschereinheit (10) geleitet werden, in weichen Füllkörper (26) aus Kieselsteinen, Lavamaterial, Kunststoffen oder anderen Körpern gefüllt sind, deren Oberfläche derart beschaffen ist, daß darauf Mikroalgen wachsen können.

14. Verfahren nach einem der Ansprüche 3 bis 12 sowie nach Anspruch 13,
**dadurch gekennzeichnet, daß** eine erste steuerbare Pumpenvorrichtung (35; 235) in dem einzigen Kreislauf bzw. im ersten Kreislauf für eine Benetzung der Füllkörper (26) mit dem Prozesswasser in Abhängigkeit von verschiedenen Parametern wie Gasmenge, Art und Menge der Inhaltsstoffe im Prozesswasser und/oder in der Abluft sorgt.

15. Verfahren nach Anspruch 4 und ggf. einem der Ansprüche 5 bis 14,
**dadurch gekennzeichnet, daß** eine zweite Pumpenvorrichtung (36) das Prozesswasser im zweiten Kreislauf und ggf. auch im ersten Kreislauf intervallartig oder kontinuierlich umwälzt.

16. Verfahren nach einem der vorhergehenden Ansprüche,
**dadurch gekennzeichnet, daß** das Prozesswasser und die Abluft die Wäschereinheit (10) im Gleichstromprinzip oder im Gegenstromprinzip durchlaufen.

17. Verfahren nach Anspruch 16,
**dadurch gekennzeichnet, daß** das Prozesswasser im Gegenstrom zu der Abluft durch eine als Gegenstrom-Sprühwäscher ohne Füllkörper ausgebildete Wäschereinheit (10) geleitet wird.

18. Verfahren nach einem der vorhergehenden Ansprüche,
**dadurch gekennzeichnet, daß** das Prozesswasser durch mindestens einen die Konversionsvorrichtung (40) bildenden Photobioreaktor (42) geleitet wird, in welchem die Algen durch Lichteinfluß Inhaltsstoffe aus dem Prozesswasser unter Erzeugung von Sauerstoff und Kohlendioxid-Aufnahme aus dem Prozesswasser einbauen.

19. Verfahren nach einem der vorhergehenden Ansprüche,
**dadurch gekennzeichnet, daß** Reinigungsgranulat dem zweiten Kreislauf zugesetzt wird, welches eine Ablagerung von Algen an Rohrinnenwänden des zweiten Kreislaufes verhindert.

20. Verfahren nach Anspruch 19,
**dadurch gekennzeichnet, daß** das Reinigungsgranulat mittels Filter vom Eindringen in den ersten Kreislauf abgehalten wird.

21. Verfahren nach einem der vorhergehenden Ansprüche,
**dadurch gekennzeichnet, daß** mittels einer Meß- und Regelungseinrichtung (80) Messungen von Parametern des Prozesswassers, wie pH-Wert, optischer Dichte, Sauerstoffkonzentration, elektrischer Leitfähigkeit, Temperatur, Volumenteilströme, durchgeführt werden und auf Basis dieser Meßwerte einzelne Verfahrensschrirte geregelt werden.

22. Verfahren nach einem der vorhergehenden Ansprüche,
**dadurch gekennzeichnet, daß** der pH-Wert des Prozesswassers mittels einer externen Kohlendioxid-Quelle (90) reguliert wird.

23. Vorrichtung zur Reduzierung der Inhaltstoffe Ammoniak, Phosphat, Ammonium, Nitrat, Nitrit, Amine, Schwefelwasserstoff, Alkohole, Ketone, Ester, organische Säuren, organische Schwefelverbindungen, aromatische Kohlenwasserstoffe und sonstige Kohlenwasserstoffverbindungen und/oder Kohlendioxid in der Abluft und im Prozesswasser einer Anlage, insbesondere zur Durchführung des Verfahrens gemäß mindestens einem der Ansprüche 4 bis 9, mit einer Wäschereinheit (10) zur Aufnahme der Inhaltstoffe aus der Abluft durch das Prozesswasser, wobei das Prozesswasser und die Abluft durch die Wäschereinheit (10) leitbar und in der Wäschereinheit (10) miteinander in Berührung bringbar sind, einer mit der Wäschereinheit (10) verbundenen, Mikroalgen enthaltenden Konversionsvorrichtung (40) zur zumindest teilweisen photosynthetischen Aufnahme von Inhaltstoffen aus dem mit Inhaltstoffen angereicherten Prozesswasser durch die Mikroalgen und einem die Wäschereinheit (10) umfassenden ersten Kreislauf für das Prozesswasser, in welchem die Abluft mit dem Prozesswasser in Kontakt kommt, und einem zumindest einen Teil der Wäschereinheit (10) und die Konversionsvorrichtung (40) umfassenden, zweiten Kreislauf für das Prozesswasser, in welchem die Mikroalgen Inhaltsstoffe aus dem Prozesswasser aufnehmen, wobei der erste Kreislauf die Wäschereinheit (10), einen in die Wäschereinheit (10) führenden ersten Einlaß (12) und mit einem Durchmischungsabschnitt (21) der Wäschereinheit (10) verbundenen ersten Auslaß (13) sowie eine den ersten Einlaß (12) und den ersten Auslaß (13) verbindende erste Verbindungsleitung (28) umfaßt, und der zweite Kreislauf den Durchmischungsabschnitt (21) der Wäschereinheit (10), einen mit diesem Durchmischungsabschnitt verbundenen zweiten Einlaß (15) und einen zweiten Auslaß (13), die Konversionsvorrichtung (40) sowie eine zweite Verbindungsleitung (29) zwischen dem zweiten Auslaß (13) und der Konversionsvorrichtung (40) und eine dritte Verbindungsleitung (30) zwischen der Konversionsvorrichtung (40) und dem zweiten Einlaß (15) umfaßt, und einer der Konversionsvorrichtung (40) nachgeschalteten Separationseinrichtung (60) zum zumindest teilweise Trennen der Mikroalgen vom Prozesswasser.

24. Vorrichtung nach Anspruch 23.
**gekennzeichnet durch** Mittel (30) zum Zurückführen des inhaltsstoffreduzierten Prozesswassers von der Konversionsvorrichturig (40) zur Wäschereinheit (10).

25. Vorrichtung nach Anspruch 23 oder 24,
**gekennzeichnet durch** ein im Durchmischungsabschnitt der Wäschereinheit (10) angeordnetes Reservoir (21), in welchem sich Prozesswasser aus beiden Kreisläufen sammelt.

26. Vorrichtung nach Anspruch 25,
**dadurch gekennzeichnet, daß** die Separationseinrichtung (60) an eine Überlaufeinrichtung (62) zur Abführung von überschüssigem Prozesswasser aus dem Reservoir (21) der Wäschereinheit (10) angeschlossen ist.

27. Vorrichtung nach einem der Ansprüche 23 bis 26,
**gekennzeichnet durch** eine Zulaufeinrichtung oder einen Vorratsbehälter (70) mit einer Zuführleitung (72) zum Zuführen von Mikroalgen und/oder Inhaltsstoffen für die Mikroalgen in den ersten und/oder zweiten Kreislauf.

28. Vorrichtung nach einem der Ansprüche 23 bis 27,
**dadurch gekennzeichnet, daß** die Wäschereinheit (10) von mindestens einem im Gleichstrom- oder Gegenstromprinzip arbeitenden Rieselbettreaktor (10) gebildet ist, in den Füllkörper (26) aus Kieselsteinen Lavamaterial, Kunststoffen oder anderen Körpern mit großen Oberflächen eingefüllt sind.

29. Vorrichtung nach einem der Ansprüche 23 bis 27,
**dadurch gekennzeichnet, daß** die Wäschereinheit (10) einen Gegenstrom-Sprühwäscher ohne Füllkörper aufweist.

30. Vorrichtung nach einem der Ansprüche 23 bis 29,
**gekennzeichnet durch** eine erste steuerbare Pumpenvorrichtung (35) im ersten Kreislauf zur Umwälzung des Prozesswassers bzw. abluftabhängigen Benetzung der Füllkörper (26) mit Prozesswasser.

31. Vorrichtung nach einem der Ansprüche 23 bis 30,
**gekennzeichnet durch** eine zweite Pumpenvorrichtung (36) zur intervallartigen oder kontinuierlichen Umwälzung des Prozesswassers im zweiten Kreislauf und ggf. auch im ersten Kreislauf.

32. Vorrichtung nach einem der Ansprüche 23 bis 31,
**dadurch gekennzeichnet, daß** die Separationseinrichtung (60) eine Zentrifuge und/oder eine Eindickvorrichtung umfaßt.

33. Vorrichtung nach einem der Ansprüche 23 bis 32,
**dadurch gekennzeichnet, daß** die Konversionsvorrichtung .(40) mindestens einen Photobioreaktor (42) aufweist.

34. Vorrichtung nach einem der Ansprüche 23 bis 33,
**dadurch gekennzeichnet, daß** die Konversionsvorrichtung ein offenes Algenkultivationssystem aufweist.

35. Vorrichtung nach einem der Ansprüche 23 bis 34,
**gekennzeichnet durch** eine Meß- und Regelungsvorrichtung (80) zur Messung von Parametern des Prozesswassers, wie pH-Wert, optischer Dichte, Sauerstoffkonzentration, elektrischer Leitfähigkeit, Temperatur, Volumenteilströme, und zur Steuerung oder Regelung einzelner Verfahrensschritte auf Basis dieser Meßwerte.

36. Vorrichtung nach einem der Ansprüche 23 bis 35,
**gekennzeichnet durch** eine externe Kohlendioxid-Quelle (90) zur Regulierung des pH-Wertes des Prozesswassers.

## Claims

1. Method of reducing the concentration of the substances ammonia, phosphate, ammonium, nitrate, nitrite, hydrogen sulphide, alcohols, ketones, esters, organic acids, organic sulphur compounds, aromatic hydrocarbons and other hydrocarbon compounds and/or carbon dioxide contained in the exhaust air and process water of a plant, comprising the steps of
a) directing the process water and exhaust air through a washer unit (10), during which the process water absorbs the substances from the exhaust air,
b) at least some of the process water containing the increased concentration of substances is introduced into a conversion device (40) containing micro-algae, in which the substances are at least partially absorbed by the micro-algae due to photosynthetic activation and
c) the micro-algae, having absorbed the substances, are at least partially separated from the process water.

2. Method as claimed in claim 1,
**characterised in that**, having had the concentration of substances contained in it reduced whilst circulating through the conversion device (40), the process water is at least partially fed back into the washer unit (10).

3. Method as claimed in claim 1 or 2,
**characterised in that** the process water is directed from the outlet (13) of the washer unit (10) to the conversion device (40) through a single circuit and, having circulated through the conversion device (40), back to an inlet (212) of the washer unit (10).

4. Method as claimed in claim 1 or 2,
**characterised in that** process water is recirculated from a first outlet (13) of the washer unit (10) to a first inlet (12) of the washer unit (10) through a first circuit, process water is directed from a second outlet (13) of the washer unit (10) to the conversion device (40) through a second circuit and, having circulated through the conversion device (40), to a second inlet (15) of the washer unit (10), and the quantities of liquid of the two circuits are mixed at least in one mixing portion (21) of the washer unit (10).

5. Method as claimed in claim 4,
**characterised in that**, having absorbed substances from the gas, at least some of the process water is at least partially collected in a mixing portion of the washer unit (10) serving as a reservoir (21) where the quantities of liquid from the first and second circuit are mixed, and process water is fed from the reservoir (21) through the first outlet (13) into the first circuit and through the second outlet (13) into the second circuit.

6. Method as claimed in claim 4 or 5,
**characterised in that** the first outlet (13) and the second outlet (13) constitute a common outlet (13).

7. Method as claimed in claim 6,
**characterised in that** a line (27) runs out from a common outlet (13) of the washer unit (10) and branches off into a first connecting line (128) of the first circuit in order to direct a part-flow of liquid to a first inlet (12) of the washer unit (10) and into a second connecting line (129) of the second circuit in order to direct a part-flow of liquid to the conversion device (40).

8. Method as claimed in one of claims 4 to 7,
**characterised in that** the process water creates an eddy as it is fed from the conversion device (40) back through the second inlet (15) into the reservoir (21) of the washer unit (10), thereby preventing the outlets (13) from being blocked by the micro-algae in the liquid.

9. Method as claimed in one of claims 4 to 8,
**characterised in that** surplus process water in the mixing portion (21) is discharged through a third outlet (18) in the washer unit (10), preferably by means of an overflow system (62), into a separation unit (60) in which the micro-algae are separated from the process water.

10. Method as claimed in one of claims 3 to 9,
**characterised in that** the quantity of substances which can be absorbed by the micro-algae and/or the quantity of micro-algae in the single circuit respectively in the first and/or second circuit is increased by an external intake.

11. Method as claimed in one of claims 3 to 10,
**characterised in that** liquid containing substances which can be absorbed by the micro-algae is fed from a supply container (70) or an intake system to the single circuit respectively to the first and/or second circuit.

12. Method as claimed in one of claims 3 to 11,
**characterised in that** the process water circulating in the single circuit respectively in the two circuits contains micro-algae.

13. Method as claimed in one of the preceding claims,
**characterised in that** the process water and/or the exhaust air is or are directed through the washer unit (10) which is provided in the form of a gravel bed reactor (10), which is packed with bodies (26) of gravel stones, lava material, synthetic materials or other bodies, the surface of which is such that micro-algae can be grown on it.

14. Method as claimed in one of claims 3 to 12 as well as claim 13,
**characterised in that** a first controllable pump system (35; 235) in the single circuit respectively in the first circuit ensures that the packing bodies (26) are wetted by the process water as a function of different parameters such as gas quantity, nature and quantity of substances in the process water and/or in the exhaust air.

15. Method as claimed in claim 4 and optionally one of claims 5 to 14,
**characterised in that** a second pump system (36) circulates the process water at intervals or continuously through the second circuit and optionally also the first circuit.

16. Method as claimed in one of the preceding claims,
**characterised in that** the process water and the exhaust air circulate through the washer unit (10) in the same flow direction or in counterflow.

17. Method as claimed in claim 16,
**characterised in that** the process water is circulated through a washer unit (10) in the form of a counterflow spray washer containing no packing bodies in the direction opposite that of the exhaust air.

18. Method as claimed in one of the preceding claims,
**characterised in that** the process water is directed through at least one photo-bioreactor (42) serving as the conversion device (40), in which the algae absorb substances from the process water under the effect of light whilst generating oxygen and absorbing carbon dioxide from the process water.

19. Method as claimed in one of the preceding claims,
**characterised in that** cleaning granulate is added to the second circuit, which prevents algae from being deposited on pipe internal walls of the second circuit.

20. Method as claimed in claim 19,
**characterised in that** the cleaning granulate is held back by filters to prevent it from penetrating the first circuit.

21. Method as claimed in one of the preceding claims,
**characterised in that** measurements of parameters of the process water such as pH value, optical density, oxygen concentration, electrical conductivity, temperature, volumes of part-flows are taken by means of a measuring and regulating system (80) and individual method steps are regulated on the basis of these measurement values.

22. Method as claimed in one of the preceding claims,
**characterised in that** the pH value of the process water is regulated by means of an external carbon dioxide source (90).

23. Device for reducing the substances ammonia, phosphate, ammonium, nitrate, nitrite, amines, hydrogen sulphide, alcohols, ketones, esters, organic acids, organic sulphur compounds, aromatic hydrocarbons and other hydrocarbon compounds and/or carbon dioxide contained in the exhaust air and process water of a plant, in particular for implementing the method as claimed in at least one of claims 4 to 9, comprising
a washer unit (10) for absorbing the substances from the exhaust air by means of the process water by means of which the process water and exhaust air can be fed through the washer unit (10) and placed in contact with one another in the washer unit (10), a conversion device (40) containing micro-algae connected to the washer unit (10) for at least partially absorbing substances from the process water containing an increased concentration of substances by means of the micro-algae due to photosynthesis, and a first circuit for the process water incorporating the washer unit (10) in which the exhaust air comes into contact with the process water, and a second circuit incorporating at least a part of the washer unit (10) and the conversion device (40) in which the micro-algae absorb substances from the process water, and the first circuit comprises the washer unit (10), a first inlet (12) leading into the washer unit (10) and a first outlet (13) connected to a mixing portion (21) of the washer unit (10) as well as a first connecting line (28) connecting the first inlet (12) and the first outlet (13), and the second circuit comprises the mixing portion (21) of the washer unit (10), a second inlet (15) connecting into this mixing portion and a second outlet (13), the conversion device (40) as well as a second connecting line (29) between the second outlet (13) and the conversion device (40) and a third connecting line (30) between the conversion device (40) and the second inlet (15), and a separation unit (60) connected downstream of the conversion device (40) for at least partially separating the micro-algae from the process water.

24. Device as claimed in claim 23,
**characterised by** means (30) for directing the process water with a reduced concentration of substances from the conversion device (40) back to the washer unit (10).

25. Device as claimed in claim 23 or 24,
**characterised by** a reservoir (21) disposed in the mixing portion of the washer unit (10) in which the process water from both circuits collects.

26. Device as claimed in claim 25,
**characterised in that** the separation unit (60) is connected to an overflow system (62) for discharging surplus process water from the reservoir (21) of the washer unit (10).

27. Device as claimed in one of claims 23 to 26,
**characterised by** an intake system or a supply container (70) with an inlet line (72) for feeding micro-algae and/or substances for the micro-algae into the first and/or second circuit.

28. Device as claimed in one of claims 23 to 27,
**characterised in that** the washer unit (10) is at least one gravel bed reactor (10) operating on the principle of a flow in the same direction or in counterflow, which is packed with bodies (26) of gravel stones, lava material, synthetic materials or other bodies with large surfaces.

29. Device as claimed in one of claims 23 to 27,
**characterised in that** the washer unit (10) is a counterflow spray washer containing no packing bodies.

30. Device as claimed in one of claims 23 to 29,
**characterised by** a first controllable pump system (35) in the first circuit for circulating the process water and wetting the packing bodies (26) with process water as a function of the exhaust air.

31. Device as claimed in one of claims 23 to 30,
**characterised by** a second pump system (36) for circulating the process water at intervals or continuously through the second circuit and optionally also through the first circuit.

32. Device as claimed in one of claims 23 to 31,
**characterised in that** the separation unit (60) comprises a centrifuge and/or a concentrator device.

33. Device as claimed in one of claims 23 to 32,
**characterised in that** the conversion device (40) has at least one photo-bioreactor (42).

34. Device as claimed in one of claims 23 to 33,
**characterised in that** the conversion device has an open algae cultivation system.

35. Device as claimed in one of claims 23 to 34,
**characterised by** a measuring and regulating system (80) for measuring parameters of the process water such as pH value, optical density, oxygen concentration, electrical conductivity, temperature, volumes of part-flows, and for controlling or regulating individual method steps on the basis of these measurement values.

36. Device as claimed in one of claims 23 to 35,
**characterised by** an external carbon dioxide source (90) for regulating the pH value of the process water.

## Revendications

1. Procédé de réduction des composants ammoniaque, phosphate, ammonium, nitrate, nitrite, amine, sulfure d'hydrogène, alcools, cétones, esters, acides organiques, composés organiques du soufre, hydrocarbures aromatiques et autres composés d'hydrocarbures et/ou dioxyde de carbone dans les effluents gazeux et dans l'eau de traitement d'une installation, comportant les étapes consistant à
a) conduire l'eau de traitement et les effluents gazeux dans une unité de lavage (10), l'eau de traitement absorbant les composants des effluents gazeux,
b) acheminer l'eau de traitement enrichie en composants au moins partiellement dans un dispositif de conversion (40) contenant des micro-algues, dans lequel les composants sont absorbés au moins en partie par les micro-algues sur la base d'une activation photosynthétique, et
c) séparer les micro-algues après absorption des composants au moins en partie de l'eau de traitement.

2. Procédé selon la revendication 1, **caractérisé en ce que** l'eau de traitement à teneur réduite en composants après avoir traversé le dispositif de conversion (40) est réacheminée au moins en partie dans l'unité de lavage (10).

3. Procédé selon la revendication 1 ou 2,
**caractérisé en ce que** l'eau de traitement est conduite dans un circuit unique d'une évacuation (13) de l'unité de lavage (10) au dispositif de conversion (40) et est réacheminée après avoir traversé le dispositif de conversion (40) à une admission (212) de l'unité de lavage (10).

4. Procédé selon la revendication 1 ou 2,
**caractérisé en ce que** l'eau de traitement est réacheminée dans un premier circuit d'une première évacuation (13) de l'unité de lavage (10) à une première admission (12) de l'unité de lavage (10), l'eau de traitement est conduite dans un deuxième circuit d'une deuxième évacuation (13) de l'unité de lavage (10) au dispositif de conversion (40) et est réacheminée après avoir traversé le dispositif de conversion (40) à une deuxième admission (15) de l'unité de lavage (10) et **en ce que** les quantités de liquides des deux circuits se mélangent au moins dans une partie de mélangeage (21) de l'unité de lavage (10).

5. Procédé selon la revendication 4, **caractérisé en ce que** l'eau de traitement est recueillie après enrichissement avec les composants du gaz, au moins en partie dans une partie de mélangeage de l'unité de lavage (10) servant de réservoir (21), dans lequel les quantités de liquide des premier et deuxième circuits se mélangent et l'eau de traitement du réservoir (21) est conduite par la première évacuation (13) dans le premier circuit et par la deuxième évacuation (13) dans le deuxième circuit.

6. Procédé selon la revendication 4 ou 5,
**caractérisé en ce que** la première évacuation (13) et la deuxième évacuation (13) forment une évacuation commune (13).

7. Procédé selon la revendication 6, **caractérisé en ce qu'**une conduite (27) part d'une évacuation commune (13) de l'unité de lavage (10), qui se divise en une première conduite de liaison (128) du premier circuit pour acheminer un courant de liquide à une première admission (12) de l'unité de lavage (10) et en une deuxième conduite de liaison (129) du deuxième circuit pour acheminer un courant de liquide au dispositif de conversion (40).

8. Procédé selon l'une des revendications 4 à 7,
**caractérisé en ce que** l'eau de traitement du dispositif de conversion (40) est réacheminée par la deuxième admission (15) en formant des remous dans le réservoir (21) de l'unité de lavage (10) de telle sorte qu'une obturation des évacuations (13) par les micro-algues dans le liquide soit empêchée.

9. Procédé selon l'une des revendications 4 à 8,
**caractérisé en ce que** l'eau de traitement excédentaire est évacuée dans la partie de mélangeage (21) par une troisième évacuation (18) dans l'unité de lavage (10), de préférence par le biais d'un dispositif de débordement (62), dans un dispositif de séparation (60), dans lequel les micro-algues sont séparées de l'eau de traitement.

10. Procédé selon l'une des revendications 3 à 9,
**caractérisé en ce que** les quantités absorbables par les micro-algues et/ou la quantité de micro-algues dans le circuit unique ou dans le premier et/ou le deuxième circuit, sont accrues par un apport externe.

11. Procédé selon l'une des revendications 3 à 10,
**caractérisé en ce que** le liquide comportant les composants absorbables par les micro-algues est acheminé d'un réservoir (70) ou d'un dispositif d'acheminement au circuit unique ou au premier et/ou au deuxième circuit.

12. Procédé selon l'une des revendications 3 à 11,
**caractérisé en ce que** l'eau de traitement circulant dans le circuit unique ou dans les deux circuits contient des micro-algues.

13. Procédé selon l'une des revendications précédentes, **caractérisé en ce que** l'eau de traitement et/ou les effluents gazeux sont acheminés dans l'unité de lavage (10) configurée comme un réacteur à lit fluidisé (10), dans lequel des corps de remplissage (26) constituées de galets, de matériaux à base de lave, de matières plastiques ou d'autres corps sont présents, dont la surface est conçue de telle sorte qu'elle permette la croissance de micro-algues sur celle-ci.

14. Procédé selon l'une des revendications 3 à 12, et selon la revendication 13, **caractérisé en ce qu'**un premier dispositif de pompage commandable (35 ; 235) dans le circuit unique ou dans le premier circuit, assure un mouillage des corps de remplissage (26) avec l'eau de traitement, en fonction de divers paramètres tels que la quantité de gaz, le type et la nature des composants dans l'eau de traitement et/ou dans les effluents gazeux.

15. Procédé selon la revendication 4 ou éventuellement, l'une des revendications 5 à 14, **caractérisé en ce qu'**un deuxième dispositif de pompage (36) recycle l'eau de traitement dans le deuxième circuit et éventuellement aussi dans le premier circuit par intervalle ou en continu.

16. Procédé selon l'une des revendications précédentes, **caractérisé en ce que** l'eau de traitement et l'effluent gazeux traversent l'unité de lavage (10) dans le sens du courant ou à contre-courant.

17. Procédé selon la revendication 16, **caractérisé en ce que** l'eau de traitement (10) est conduite à contre-courant aux effluents gazeux par une unité de lavage (10) conçue comme un dispositif de lavage à pulvérisation à contre-courant, sans corps de remplissage.

18. Procédé selon l'une des revendications précédentes, **caractérisé en ce que** l'eau de traitement est conduite par au moins un photobioréacteur (42) formant le dispositif de conversion (40), dans lequel les algues introduisent sous l'effet de la lumière, des composants de l'eau de traitement en produisant de l'oxygène et en absorbant du dioxyde de carbone.

19. Procédé selon l'une des revendications précédentes, **caractérisé en ce que** le granulat de purification est ajouté au deuxième circuit, qui empêche un dépôt des algues sur les parois internes des tubes du deuxième circuit.

20. Procédé selon la revendication 19, **caractérisé en ce qu'**un filtre empêche le granulat de purification de pénétrer dans le premier circuit.

21. Procédé selon l'une des revendications précédentes, **caractérisé en ce que**, au moyen d'un dispositif de mesure et de régulation (80), on réalise des mesures des paramètres de l'eau de traitement, tels que le pH, la densité optique, la concentration en oxygène, la conductivité électrique, la température, les courants volumiques partiels et à base de ces mesures, on régule des étapes individuelles du procédé.

22. Procédé selon l'une des revendications précédentes, **caractérisé en ce que** le pH de l'eau de traitement est régulé à l'aide d'une source de dioxyde de carbone externe (90).

23. Dispositif de réduction des composants ammoniaque, phosphate, ammonium, nitrate, nitrite, amine, sulfure d'hydrogène, alcools, cétones, esters, acides organiques, composés organiques du soufre, hydrocarbures aromatiques et autres composés d'hydrocarbures et/ou dioxyde de carbone dans les effluents gazeux et dans l'eau de traitement d'une installation, en particulier pour la réalisation du procédé selon au moins l'une des revendications 4 à 9, avec une unité de lavage (10) pour absorber les composants des effluents gazeux par l'eau de traitement, l'eau de traitement et les effluents gazeux pouvant être conduits dans l'unité de lavage (10) et pouvant être mis en contact les uns avec les autres dans l'unité de lavage (10), avec un dispositif de conversion (40) contenant des micro-algues liées à l'unité de lavage (10) pour au moins l'absorption photosynthétique partielle de composants de l'eau de traitement enrichie avec les composants par les micro-algues, et un premier circuit comprenant l'unité de lavage (10) pour l'eau de traitement, dans lequel les effluents gazeux entrent en contact avec l'eau de traitement et un deuxième circuit comprenant au moins une partie de l'unité de lavage (10) et le dispositif de conversion (40), pour l'eau de traitement, dans lequel les micro-algues absorbent des composants, le premier circuit comprenant l'unité de lavage (10), une première admission (12) conduisant à l'unité de lavage (10) et avec une première évacuation (13) reliée à une partie de mélangeage (21) de l'unité de lavage (10) et une première conduite de liaison (28) reliant l'une des première admission (12) et première évacuation (13) et le deuxième circuit comprenant la partie de mélangeage (21) de l'unité de lavage (10), une deuxième admission (15) reliée à cette partie de mélangeage et une deuxième évacuation (13), le dispositif de conversion (40) et une deuxième conduite de liaison (29) entre la deuxième évacuation (13) et le dispositif de conversion (40) et une troisième conduite de liaison (30) entre le dispositif de conversion (40) et la deuxième admission (15), et avec un dispositif de séparation (60) en amont du dispositif de conversion (40) pour séparer au moins en partie les micro-algues de l'eau de traitement.

24. Dispositif selon la revendication 23, **caractérisé par** un moyen (30) de réacheminement de l'eau de traitement à teneur réduite en composants du dispositif de conversion (40) à l'unité de lavage (10).

25. Dispositif selon la revendication 23 ou 24, **caractérisé par** un réservoir (21) disposé dans une partie de mélangeage de l'unité de lavage (10), dans lequel l'eau de traitement des deux circuits s'accumule.

26. Dispositif selon la revendication 25, **caractérisé en ce que** le dispositif de séparation (60) est raccordé à un dispositif de déversement (62) pour évacuer l'eau de traitement excédentaire du réservoir (21) de l'unité de lavage (10).

27. Dispositif selon l'une des revendications 23 à 26, **caractérisé par** un dispositif d'acheminement ou un réservoir (70) comportant une conduite d'acheminement (72) pour acheminer des micro-algues et/ou des composants pour les micro-algues dans le premier et/ou le deuxième circuit.

28. Dispositif selon l'une des revendications 23 à 27, **caractérisé en ce que** l'unité de lavage (10) est formée d'au moins un réacteur à lit fluidisé (10) fonctionnant dans le sens du courant ou à contre-courant, dans lequel sont placés les corps de remplissage (26) en matériau à base de lave, en matières plastiques ou autres corps présentant une grande surface.

29. Dispositif selon l'une des revendications 23 à 27, **caractérisé en ce que** l'unité de lavage (10) présente un dispositif de lavage par pulvérisation à contre-courant sans corps de remplissage.

30. Dispositif selon l'une des revendications 23 à 29, **caractérisé par** un premier dispositif de pompage (35) commandable dans le premier circuit pour le recyclage de l'eau de traitement ou le mouillage des corps de remplissage (26), en fonction des effluents gazeux, avec l'eau de traitement.

31. Dispositif selon l'une des revendications 23 à 30, **caractérisé par** un deuxième dispositif de pompage (36) pour le recyclage par intervalle ou en continu de l'eau de traitement dans le deuxième circuit et éventuellement aussi dans le premier circuit.

32. Dispositif selon l'une des revendications 23 à 31, **caractérisé en ce que** le dispositif de séparation (60) comprend une centrifugeuse et/ou un dispositif de condensation.

33. Dispositif selon l'une des revendications 23 à 32, **caractérisé en ce que** le dispositif de conversion (40) présente au moins un photobioréacteur (42).

34. Dispositif selon l'une des revendications 23 à 33, **caractérisé en ce que** le dispositif de conversion présente un système de culture des algues.

35. Dispositif selon l'une des revendications 23 à 34, **caractérisé par** un dispositif de mesure et de régulation (80) pour mesurer des paramètres de l'eau de traitement, tels que le pH, la densité optique, la concentration en oxygène, la conductivité électrique, la température, les courants volumiques partiels et pour réguler, à base de ces mesures, des étapes individuelles du procédé.

36. Dispositif selon l'une des revendications 23 à 35, **caractérisé par** une source externe de dioxyde de carbone (90) pour réguler le pH de l'eau de traitement.
